# EUROPEAN PATENT APPLICATION

(11) **EP 4 238 982 A1**
(43) Date of publication of application: **06.09.2023**
(21) Application number: 21863087.9
(22) Date of filing: 18.08.2021
(51) Int. Cl.: C07K 7/08, A61K 38/08, A61K 47/65, A61P 35/00, C07K 14/47, C07K 14/82, C12N 15/00

(54) **ANTI-TUMOR PEPTIDE AND USE THEREOF**

(30) Priority: 28.10.2020 JP 2020180811
(71) Applicant: NATIONAL UNIVERSITY CORPORATION HOKKAIDO UNIVERSITY, Kita-ku Sapporo-shi Hokkaido 060-0808 (JP)
(72) Inventor: MATSUDA, Tadashi, Hokkaido 0600808 (JP); ORITANI, Kenji, Chiba 2860124 (JP); KITAI, Yuichi, Hokkaido 0600808 (JP)
(74) Representative: Prüfer & Partner mbB Patentanwälte · Rechtsanwälte
(86) International application number: PCT/JP2021/030265
(87) International publication number: WO 2022/091531

(57) **Abstract**

The present invention provides a peptide that consists of an amino acid sequence with 20 or fewer residues containing at least one amino acid sequence selected from the group consisting of the amino acid sequence represented in SEQ ID NO: 1, the amino acid sequence represented in SEQ ID NO: 2 and the amino acid sequence represented in SEQ ID NO: 38, the peptide having an activity to inhibit proliferation of a tumor cell or having an activity to inhibit binding between an epidermal growth factor receptor and a signal-transducing adaptor family member-2 (STAP-2); a peptide with a cell-penetrating peptide added to a terminus of the above peptide with or without a linker sequence interposed between the cell-penetrating peptide and the terminus; and pharmaceutical compositions containing any of these peptides.

## Description

### FIELD

The present invention relates to a peptide having an antitumor activity, and a pharmaceutical composition containing the peptide.

### BACKGROUND

Signal transducing adaptor protein-2 (STAP-2) is an adaptor protein that has, starting from the N-terminus, a PH domain capable of binding to phospholipids, an SH2-like domain corresponding to a phosphotyrosine-binding region, and a proline-rich region capable of binding to an SH3 domain-containing protein. STAP-2 has been observed to be highly expressed in various types of cancers such as breast cancer and prostate cancer. Non Patent Literatures 1 to 3 report that STAP-2 interacts with the breast cancer-specific tyrosine kinase Brk (Breast tumor kinase) and contributes to activation of STAT3, and that genetic knockdown of STAP-2 inhibits proliferation of breast cancer cells and prostate cancer cells. Further, Non Patent Literatures 4 and 5 report that STAP-2 enhances the activity of BCR-ABL tyrosine kinase, which is responsible for chronic myelogenous leukemia (CML), thereby enhancing BCR-ABL-dependent cell proliferation, and that the genetic knockdown of STAP-2 inhibits tumorigenesis of human CML cancer cells.

Based on these findings, inhibition of STAP-2 function is expected to be a new approach to cancer therapy.

### CITATION LIST

### NON PATENT LITERATURES

Non Patent Literature 1: J. Biol. Chem. 285: 38093-38103, 2010
Non Patent Literature 2: Cancer Sci. 102: 756-761, 2011
Non Patent Literature 3: J. Biol. Chem. 292: 19392-19399, 2017
Non Patent Literature 4: Oncogene 26: 6038-6049, 2012
Non Patent Literature 5: Biochem. Biophys. Res. Commun. 463: 825-831, 2015

### SUMMARY

### TECHNICAL PROBLEM

The present invention is aimed at providing a new substance having an inhibitory effect on proliferation of tumor cells.

### SOLUTION TO PROBLEM

Inventors of the present invention have found that STAP-2 promotes proliferation of prostate cancer cells through upregulation of epidermal growth factor receptor (EGFR) signaling (Non Patent Literature 3). Therefore, the inventors focused their research on regulating proliferation of cancer cells by control of EGFR/STAP-2 interaction, and found that a peptide containing a specific partial amino acid sequence in the PH domain of STAP-2 can inhibit proliferation of tumor cells, and completed the following invention.
item 1. A peptide consisting of an amino acid sequence with 20 or fewer residues containing at least one amino acid sequence selected from the group consisting of the amino acid sequence represented in SEQ ID NO: 1, the amino acid sequence represented in SEQ ID NO: 2, and the amino acid sequence represented in SEQ ID NO: 38, the peptide having an activity to inhibit proliferation of a tumor cell.
item 2. The peptide according to item 1, consisting of an amino acid sequence with 20 or fewer residues containing the amino acid sequence represented in SEQ ID NO: 3.
item 3. The peptide according to item 1 or 2, consisting of an amino acid sequence with 20 or fewer residues containing the amino acid sequence represented in SEQ ID NO: 4.
item 4. The peptide according to item 1, wherein 3rd amino acid residue Xaa is isoleucine, alanine, leucine or valine, and 4th amino acid residue Xaa is tyrosine, alanine or phenylalanine, in the amino acid sequence represented in SEQ ID NO: 38.
item 5. A peptide consisting of an amino acid sequence with 20 or fewer residues containing at least one amino acid sequence selected from the group consisting of the amino acid sequence represented in SEQ ID NO: 1, the amino acid sequence represented in SEQ ID NO: 2 and the amino acid sequence represented in SEQ ID NO: 38, the peptide having an activity to inhibit binding between an epidermal growth factor receptor and a signal-transducing adaptor family member-2 (STAP-2).
item 6. The peptide according to item 5, consisting of an amino acid sequence with 20 or fewer residues containing the amino acid sequence represented in SEQ ID NO: 3.
item 7. The peptide according to item 5 or 6, consisting of an amino acid sequence with 20 or fewer residues containing the amino acid sequence represented in SEQ ID NO: 4.
item 8. The peptide according to item 5, wherein 3rd amino acid residue Xaa is isoleucine, alanine, leucine or valine, and 4th amino acid residue Xaa is tyrosine, alanine or phenylalanine, in the amino acid sequence represented in SEQ ID NO: 38.
item 9. A peptide with a cell-penetrating peptide added to a terminus of the peptide according to any one of items 1 to 8, with or without a linker sequence interposed between the cell-penetrating peptide and the terminus.
item 10. The peptide according to item 9, wherein the linker sequence consists of one to five glycine(s).
item 11. The peptide according to any one of items 1 to 10, comprising one or more chemically modified amino acid residue(s).
item 12. A nucleic acid encoding the peptide according to any one of items 1 to 11.
item 13. A pharmaceutical composition for treating a cancer, the pharmaceutical composition comprising the peptide according to any one of items 1 to 11 or the nucleic acid according to item 12.
item 14. The pharmaceutical composition according to item 13, for treating prostate cancer, cervical cancer, liver cancer, lung cancer, glioma, melanoma, pancreatic cancer or breast cancer.
item 15. A composition for inhibition of binding between an epidermal growth factor receptor and a signal-transducing adaptor family member-2 (STAP-2), the composition comprising the peptide according to any one of items 1 to 11 or the nucleic acid according to item 12.

### EFFECT OF THE INVENTION

The peptide and pharmaceutical composition containing the peptide according to the present invention can be used for treating cancers.

### BRIEF DESCRIPTION OF DRAWINGS

Fig. 1 is a graph showing proliferation of human prostate cancer cell line DU145 in the presence of synthetic peptides #1 to #7. Data are represented as average value + standard error (The same is applied to all following graphs, unless otherwise specifically described).
Fig. 2 is a graph showing proliferation of DU145 cells in the presence of synthetic peptides #2-A, #2-B and #2-C.
Fig. 3 is a graph showing proliferation of DU145 cells in the presence of synthetic peptides #2-D, #2-D-1, #6-A and #6-B.
Fig. 4 is a graph showing proliferation of DU145 cells in the presence of synthetic peptides #2-D-1-1, #2-D-1-2, #2-D-1-3, #6-A, #6-A-1 and #6-A-2.
Fig. 5 is a graph showing proliferation of DU145 cells in the presence of synthetic peptides #2-D-1-4, #2-D-1-1A, #2-D-1-1B and #7-A.
Fig. 6 is a set of graphs showing proliferation of DU145 cells, human cervical cancer cell line HeLa, human liver cancer cell line Hep3B, human lung cancer cell line A549, human glioma cell line U251, human melanoma cell line A375, human pancreatic cancer cell line BxPC3 and human breast cancer cell line T47D, in the presence of synthetic peptide #2-D-1-4. n=3; *p <0.05 (paired Student's t test)
Fig. 7 is a graph showing proliferation of DU145 cells in the presence of synthetic peptides #T-2-D-1-4, #H-2-D-1-4 and #F-2-D-1-4 in which a cell-penetrating peptide is substituted in the synthetic peptide #2-D-1-4.
Fig. 8 is a graph showing proliferation of DU145 cells in the presence of peptides #R8(-)-2-D-1, #R8(-)-2-D-1-4 and #R8(-)-6-A-2, all of which have no cell-penetrating peptide.
Fig. 9 is a graph showing proliferation of DU145 cells in the presence of synthetic peptides #2-D-1-4, #2-D-1-4-1A, #2-D-1-4-2A, #2-D-1-4-3A, #2-D-1-4-4A, #2-D-1-4-5A, #2-D-1-4-6A and #2-D-1-4-7A. n=3; *p <0.05 (paired Student's t test)
Fig. 10 is a set of graphs showing expression levels of CyclinD1 (Ccnd1) gene and Survivin gene in DU145 cell in the presence of synthetic peptide #2-D-1-4.
Fig. 11 is a set of images of immunoblots after pull-down assay indicating an effect of the synthetic peptide #2-D-1-4 on interaction between EGFR and STAP-2.
Fig. 12 is a set of a graph (left) showing time-dependent changes in tumor volumes of tumor-bearing mice to which the synthetic peptide #2-D-1-4 was administered, and a plot (right) showing weights of tumor mass on 32nd day after cancer cell implantation. n=6; *p <0.05 (paired Student's t test)

### DESCRIPTION OF EMBODIMENTS

The following description of the present invention may be based on representative embodiments or specific examples, but the present invention is not limited to such embodiments or specific examples. In this description, the upper and lower limits of each numerical range can be arbitrarily combined with each other. In this description, numerical ranges represented by symbols "∼" and "-" refer to ranges in which both numerals limiting the range are included as the upper and lower limits thereof, unless otherwise specified. In principle, amino acids and amino acid residues are denoted by single letter code.

### Antitumor peptide

A first aspect of the present invention relates to a peptide that consists of an amino acid sequence with 20 or fewer residues containing at least one amino acid sequence selected from the group consisting of the amino acid sequence (GLTI) represented in SEQ ID NO: 1, the amino acid sequence (VVELRVP) represented in SEQ ID NO: 2 and the amino acid sequence (L-T-Xaa1-Xaa2-F, in which Xaa1 and Xaa2 individually represent any amino acid residues) represented in SEQ ID NO: 38. The peptide has an activity to inhibit proliferation of tumor cells (hereinafter referred to as antitumor peptide).

In an embodiment of the present invention, the antitumor peptide consists of or contains at least one of the amino acid sequence represented in SEQ ID NO: 1 and the amino acid sequence represented in SEQ ID NO: 2, both of which are partial amino acid sequences in the PH domain of human STAP-2. When containing the amino acid sequence represented in SEQ ID NO: 1, the peptide preferably consists of or contains the amino acid sequence (GLTIY) represented in SEQ ID NO: 3, more preferably consists of or contains the amino acid sequence (GLTIYF) represented in SEQ ID NO: 4.

In another embodiment of the present invention, the antitumor peptide consists of or contains the amino acid sequence represented in SEQ ID NO: 38. When containing the amino acid sequence represented in SEQ ID NO: 38, the peptide preferably consists of or contains the amino acid sequence represented in SEQ ID NO: 4.

Amino acid sequences of STAP-2 are known, and the amino acid sequence (SEQ ID NO: 5) of human STAP-2 is registered as NP_001013863.1 in the Reference Sequence Database of the National Center for Biotechnology Information (NCBI). STAP-2 is a protein having, starting from its N-terminus, a PH domain (a region from 1st to 147th residues of SEQ ID NO: 5) capable of binding to a phospholipid, an SH2-like domain (a region from 152nd to 247th residues of SEQ ID NO: 5) corresponding to a phosphotyrosine-bound region, and a proline-rich region (a region from 247th to 403rd residues of SEQ ID NO: 5) capable of binding to an SH3 domain-containing protein.

The amino acid sequence represented in SEQ ID NO: 1 corresponds to 45th to 48th residues of the amino acid sequence of SEQ ID NO: 5. The amino acid sequence represented in SEQ ID NO: 2 corresponds to 116th to 122nd residues of the amino acid sequence of SEQ ID NO: 5. The amino acid sequence represented in SEQ ID NO: 3 corresponds to 45th to 49th residues of the amino acid sequence of SEQ ID NO: 5. The amino acid sequence represented in SEQ ID NO: 4 corresponds to 45th to 50th residues of the amino acid sequence of SEQ ID NO: 5. The amino acid sequence represented by SEQ ID NO: 38 is an amino acid sequence in which amino acid residues substitutable in view of activity to inhibit proliferation of tumor cells are represented as Xaa1 and Xaa2 in the sequence from 46th to 50th residues of the amino acid sequence of SEQ ID NO: 5. Xaa1 is preferably I, A, L or V. Xaa2 is preferably Y, A or F. The amino acid sequence represented by SEQ ID NO: 38 is listed in the sequence listing as L-T-Xaa-Xaa-F, in which the 3rd residue Xaa corresponds to Xaa1 and the 4th residue Xaa corresponds to Xaa2.

In the present invention, the antitumor peptide consists of an amino acid sequence with 20 or fewer residues. In a preferred embodiment, the number of the amino acid residues in the antitumor peptide may be 15 or less, 12 or less, or 10 or less.

When the antitumor peptide contains the amino acid sequence represented in SEQ ID NO: 1, the number of amino acid residues in the antitumor peptide may be 4 or more, for example 5 or more, preferably 6 or more, 7 or more, 8 or more, or 9 or more. When the antitumor peptide contains the amino acid sequence represented in SEQ ID NO: 3, the number of amino acid residues in the antitumor peptide may be 5 or more, preferably 6 or more, 7 or more, 8 or more, or 9 or more. When the antitumor peptide contains the amino acid sequence represented in SEQ ID NO: 4, the number of amino acid residues in the antitumor peptide may be 6 or more, preferably 7 or more, 8 or more, or 9 or more. When the antitumor peptide contains the amino acid sequence represented in SEQ ID NO: 38, the number of amino acid residues in the antitumor peptide may be 5 or more, preferably 6 or more, 7 or more, 8 or more, or 9 or more.

The antitumor peptide may contain one or more amino acid sequences selected from the group consisting of the amino acid sequence represented in SEQ ID NO: 1, the amino acid sequence represented in SEQ ID NO: 2, and the amino acid sequence represented in SEQ ID NO: 38, as long as the number of amino acid residues in the antitumor peptide is 20 or less.

In the present invention, the antitumor peptide may contain any amino acid sequence other than amino acid sequences represented in one of SEQ ID NOs: 1 to 4 and 38. For example, the antitumor peptide may additionally contain a partial amino acid sequence in the amino acid sequence represented in SEQ ID NO: 5, the partial amino acid sequence being adjacent to N- or C-terminal side of the amino acid sequence represented in one of SEQ ID NOs: 1 to 4. A preferred example of the antitumor peptide is a peptide consisting of the amino acid sequence (GLTIYFY) represented in SEQ ID NO: 36. The amino acid sequence represented in SEQ ID NO: 36 is a partial sequence of the amino acid sequence represented in SEQ ID NO: 5, and is a sequence with an additional Y on the C-terminal side of the amino acid sequence represented in SEQ ID NO: 4.

The antitumor peptide has an activity to inhibit proliferation of tumor cells when coexisting with the tumor cells. Examples of the tumor cells of which proliferation to be inhibited by the antitumor peptide include cancer cells derived from prostate cancer, cervical cancer, liver cancer, lung cancer, glioma, melanoma, pancreatic cancer, and breast cancer.

### Antitumor peptide with cell-penetrating peptide added

The above antitumor peptide can be used in a form of a modified peptide in which another substance is added to the antitumor peptide as long as the activity to inhibit proliferation of tumor cells is not impaired. The modified peptide is also encompassed in the present invention.

One example of the modified peptide is a peptide in which a cell-penetrating peptide is added to the terminus of the above antitumor peptide with or without a linker sequence interposed therebetween. Thus, the present invention also provides the peptide in which a cell-penetrating peptide is added to the terminus of the above antitumor peptide with or without the linker sequence interposed therebetween.

Cell-penetrating peptides (hereinafter represented as CPPs) are peptides capable of enhancing cell membrane penetrability of a substance to which the peptides are bound, and utilized as tools for introducing various substances such as proteins, peptides, high molecular weight drugs, nanoparticles and liposomes into cells.

In the present invention, any peptide selected from known CPPs can be utilized, examples of which include an oligoarginine peptide, an arginine-rich basic peptide (TAT peptide, SEQ ID NO: 6) derived from the Tat protein of human immunodeficiency virus type 1, an arginine-rich basic peptide (HTLV-II-Rex, SEQ ID NO: 7) derived from the Rex protein of human T-cell leukemia virus type II, and an arginine-rich basic peptide (FHV coat (35-49), SEQ ID NO: 8) derived from flock house virus. The oligoarginine peptide refers to a peptide consisting of a plurality of consecutive Arg residues. The number of Arg residues in the oligoarginine peptide is approximately 4 to 16, preferably 6 to 12, more preferably 8 to 12.

CPPs can be added to either the N-terminus or C-terminus of the antitumor peptides. The CPPs can be added to either the N-terminus or C-terminus of the antitumor peptides directly or via a linker sequence.

Any linker sequence (linker peptide) selected from linker sequences known to the person skilled in the art can be utilized, as long as the linker sequence connects two peptides without interfering functions of the two peptides. The number of amino acid residues in the linker sequence is preferably 1 to 10, for example, 1 to 5, 1 to 4, 1 to 3, 1 to 2, 2 to 3, 2 to 4, 2 to 5, or 2 to 10. The amino acid residue constituting the linker sequence is preferably a relatively less bulky amino acid such as Gly, Ala, Ser and Pro, and particularly preferably Gly. In a particular embodiment, the linker sequence is a sequence consisting of 1 to 5, 1 to 4, 1 to 3, 1 to 2, 2 to 3, 2 to 4, or 2 to 5 consecutive glycine residues.

A preferred example of the peptide in which a cell-penetrating peptide is added to the antitumor peptide is a peptide in which a cell-penetrating peptide is added to the antitumor peptide via a linker sequence containing one to five glycine residue(s). A particularly preferred example of the peptide in which a cell-penetrating peptide is added to the antitumor peptide is a peptide having an amino acid sequence consisting of, starting from the N-terminus, an amino acid sequence of a cell-penetrating peptide, a linker sequence of one to five glycine residues, and the amino acid sequence represented in SEQ ID NO: 36.

### Other modified peptides

Another example of the modified peptide is the antitumor peptide with one or more amino acid residues chemically modified with a suitable substance. Examples of the chemical modification include modification of an amino group (such as biotinylation, myristoylation, palmitoylation, acetylation, maleimidation, methylation and malonylation), modification of a carboxyl group (such as amidation and esterification), modification of a thiol group (such as farnesylation, geranylation, methylation and palmitoylation), modification of a hydroxy group (such as phosphorylation and sulfation), PEGylation and glycosylation, in amino acid residues.

Another example of the modified peptide is a peptide in which a labeling compound such as a fluorescent substance is added to the antitumor peptide. Examples of the labeling compound include fluorescent substances (e.g., FITC, rhodamine and the like), metal particles (e.g., gold colloid and the like), fluorescent microbeads (e.g., Luminex (registered trademark, Luminex Corporation) and the like), pigment proteins (e.g., phycoerythrin, phycocyanin and the like), radioisotopes (e.g., ³H, ¹⁴C, ³²P, ³⁵S, ¹²⁵I, ¹³¹I and the like), enzymes (for example, peroxidase, alkaline phosphatase and the like), biotin and streptavidin.

Another example of the modified peptide is a peptide in which the antitumor peptide is fused to a functional protein. Examples of the functional protein include tag peptides such as His tag, GST tag, HA tag and FLAG tag as well as fluorescent proteins such as GFP.

### Preparation of peptide

In the present invention, peptides can be produced with use of amino acids modified with various protecting groups as raw materials, by organic chemical synthesis methods such as Fmoc method (fluorenylmethyloxycarbonyl method) and tBoc method (t-butyloxycarbonyl method), for example.

Peptides can also be produced by a genetic engineering method that involves introduction of expression vectors containing nucleic acids encoding the peptides into suitable host cells to express the peptides, or by a genetic engineering method that involves translation of nucleic acids encoding the peptides in a cell-free protein synthesis system. Each operation in the genetic engineering method including preparation of nucleic acids encoding the peptides, types of host cells, methods for gene introduction, expression and purification of the peptides and the like, can be carried out by a method known or well-known to the person skilled in the art according to instructions of experimental operation manuals that describe in detail about various genetic engineering operations. Another aspect of the present invention also provides a nucleic acid encoding the antitumor peptide, and a host cell transformed by the nucleic acid.

The nucleic acid encoding the antitumor peptide may be an mRNA encoding the antitumor peptide or an expression vector containing a DNA encoding the antitumor peptide. The expression vector is not limited in its construction as long as it can express the antitumor peptide, and typically contains a promoter and a nucleic acid sequence encoding the antitumor peptide located under the control of the promoter. The expression vector may further contain any functional nucleic acid sequence that regulates transcription and expression, such as an operator, an enhancer and the like. Any promoter and functional nucleic acid sequence appropriately selected from those can function in the cell into which the expression vector is introduced can be used.

The type of the expression vector is not particularly limited, and examples thereof include plasmid vectors, and viral vectors such as retroviral vectors, lentiviral vectors, adenoviral vectors, adeno-associated viral vectors, herpes viral vectors and Sendai viral vectors.

Examples of the host cell include bacteria such as Escherichia, Bacillus, Corynebacterium, Brevibacterium, Serratia, Pseudomonas, Arthrobacter, Erwinia, Methylobacterium and Rhodobacter, as well as fungi such as Streptomyces, Zymomonas and Saccharomyces. It is possible to use an insect cell such as silkworm cell, as well as an animal cell such as HEK293 cell, MEF cell, Vero cell, Hela cell, CHO cell, WI38 cell, BHK cell, COS-7 cell, MDCK cell, C127 cell, HKG cell, and human kidney cell line.

Examples of promoters for prokaryotic cells include lac promoter, tac promoter, tet promoter and ara promoter. Examples of promoters for eukaryotic cells include: pol II promoters such as CMV promoter, EF 1 promoter, SV40 promoter, MSCV promoter, hTERT promoter, β-actin promoter and CAG promoter; and pol III promoters such as mouse and human U6-snRNA promoters, human H1-RNase P RNA promoter and human valine-tRNA promoter.

### Composition for inhibition of EGFR-STAP-2 binding

Although not bound by any theory, the antitumor peptide has a partial amino acid sequence of the PH domain in human STAP-2, and is considered to inhibit the binding between human EGFR and STAP-2 so as to inhibit proliferation of tumor cells. Accordingly, the present invention further provides: a peptide (hereinafter referred to as a binding inhibitory peptide) that consists of an amino acid sequence with 20 or fewer residues containing at least one amino acid sequence selected from the group consisting of the amino acid sequence represented in SEQ ID NO: 1, the amino acid sequence represented in SEQ ID NO: 2 and the amino acid sequence represented in SEQ ID NO: 38 and has an activity to inhibit the binding between EGFR and STAP-2; a nucleic acid encoding the binding inhibitory peptide (encompassing an mRNA encoding the binding inhibitory peptide, and an expression vector that contains a DNA encoding the binding inhibitory peptide); a host cell transformed with the nucleic acid; and a composition for inhibition of binding between EGFR and STAP-2 comprising the binding inhibitory peptide.

### Pharmaceutical composition

The present invention further provides a pharmaceutical composition for treating a cancer, containing the antitumor peptide or the binding inhibitory peptide, or a nucleic acid encoding any of these peptides.

The pharmaceutical composition contains one or more of the antitumor peptide(s) or the binding inhibitory peptides, or nucleic acids encoding these peptides, in an amount effective for treating cancers. The antitumor peptide or the binding inhibitory peptide contained in the pharmaceutical composition may be in a form of a modified peptide in which another substance is added to the antitumor peptide or the binding inhibitory peptide. The effective amount of the antitumor peptide, the binding inhibitory peptide, or nucleic acids encoding these peptides can be determined as appropriate, depending on usages, ages of subject, sexes, weights, types of cancers and other conditions.

The term "treatment" as used herein encompasses all types of therapeutic interventions medically acceptable for purposes including curing or temporary remission of diseases. Accordingly, the treatment of cancers encompasses interventions medically acceptable for various purposes including delay or stop of the progression of cancers, retraction or disappearance of lesions, prevention of disease onset, prevention of metastasis or recurrence, and the like.

The pharmaceutical composition is administered to cancer-affected subjects, for example, mammals such as rodents including mice, rats, hamsters and guinea pigs, primates including humans, chimpanzees, and rhesus monkeys, domestic animals including pigs, cattle, goats, horses and sheep, and companion animals including dogs and cats. A preferred subject is humans.

Cancers suitable for treatment with the pharmaceutical composition are cancers involving the interaction between EGFR and STAP-2, such as prostate cancer, cervical cancer, liver cancer, lung cancer, glioma, melanoma, pancreatic cancer and breast cancer.

In addition to the antitumor peptides or the binding inhibitory peptides or the nucleic acids encoding these peptides, the pharmaceutical composition may contain another drug for treatment of cancers, or a pharmaceutically acceptable additive, for example, a buffer, a stabilizer, a preservative, an excipient, or the like. The pharmaceutically acceptable additive can be selected and used as appropriate depending on pharmaceutical formulation from components that are well known to the person skilled in the art and described in, for example, Japanese Pharmacopoeia 17th Edition and other standard documents, within the scope of normal practice capabilities for the person skilled in the art.

The dosage form of the pharmaceutical composition can be any form, and can be selected as appropriate depending on target sites and types of cancers. The dosage form of the pharmaceutical composition is preferably a parenteral formulation, and can be an injection or a drip, for example. The administration route of the pharmaceutical composition is not particularly limited. Examples of the administration for parenteral formulation include intravascular administration (preferably intravenous administration), subcutaneous administration, intramuscular administration, intraperitoneal administration, and topical administration to a target site. In one of the preferred embodiments, the pharmaceutical composition of the invention is administered to a living body by intravenous administration or topical administration.

In order to efficiently deliver the antitumor peptide or the binding inhibitory peptide into cancer cells, the pharmaceutical composition containing the antitumor peptide or the binding inhibitory peptide may be blended or administered with an agent to facilitate transfer of the peptide into cells, such as a cationic lipid, a cell-penetrating peptide that noncovalently interacts with a protein to form a complex to be transferred into cells, Sendai virus-derived envelope (HVJ-E) or a magnetic nanoparticle.

In order to efficiently deliver the nucleic acid encoding the antitumor peptide or the binding inhibitory peptide into cancer cells, the pharmaceutical composition containing the nucleic acid may be blended or administered with an agent to facilitate transfer of the nucleic acid into cells, for example a cationic lipid such as lipofectamine.

The pharmaceutical composition containing the nucleic acid is preferably administered by use of a drug delivery system (DDS) suitable for introducing nucleic acids into cancer cells. Examples of such DDS include those utilizing DDS materials including viral vectors, lipid membrane constructs such as liposomes and micelles, particularly cationic lipid membrane constructs suitable for delivery of nucleic acids into cells, and magnetic microparticles. An aspect of the present invention includes a pharmaceutical composition in a form in which the nucleic acid encoding the antitumor peptide or the binding inhibitory peptide is supported on or encapsulated in such DDS materials as well as the nucleic acid that is supported on or encapsulated in such DDS materials.

The present invention will be described in more detail with reference to the following Examples, but not limited thereto.

### EXAMPLES

### Example 1 Synthesis of peptide

The peptides listed in Table 1 were designed with reference to an amino acid sequence (amino acid sequence with 1st to 147th residues of SEQ ID NO: 5) of the PH domain of STAP-2. Each of the peptides has, starting from the N-terminal side, an oligoarginine sequence (octaarginine sequence) of eight Arg residues, a linker sequence of two Gly residues, and a partial amino acid sequence of the PH domain or a modified sequence of the partial amino acid sequence. These chemically synthesized peptides (95% or more of peptide purity) were purchased from GL Biochem, and dissolved to prepare 10% DMSO solution for use in experiments.

**[Table 1]**

| Peptide No. | Amino Acid Sequence | Partial Amino Acid Sequence of PH Domain | SEQ ID NO: |
|---|---|---|---|
| #1 | RRRRRRRRGGPSHYYESFLEKKGPCDRDYK | P18-K37 | 9 |
| #2 | RRRRRRRRGGDRDYKKFWAGLQGLTIYFYN | D33-N52 | 10 |
| #3 | RRRRRRRRGGFYNSNRDFQHVEKLNLGAFE | F50-E69 | 11 |
| #4 | RRRRRRRRGGGSSRDPGTHFSLILRDQEIK | G78-K97 | 12 |
| #5 | RRRRRRRRGGDQEIKFKVETLECREMINKGF | D93-F112 | 13 |
| #6 | RRRRRRRRGGMWKGFILT**VVELRVP**TDLTL | M108-L127 | 14 |
| #7 | RRRRRRRRGGTDLTLLPGH LYM M S EV LAKE | T123-E142 | 15 |
| #2-A | RRRRRRRRGGFWA**GLQGLTIY**FYNSNRDFQ | F39-Q58 | 16 |
| #2-B | RRRRRRRRGGFYNSNRDFQHVEKLN | F50-N64 | 17 |
| #2-C | RRRRRRRRGGQ**GLTIYF**YNSNRDFQ | Q44-Q58 | 18 |
| #2-D | RRRRRRRRGGQ**GLTIYFY**FNSNR | Q44-R55 | 19 |
| #2-D-1 | RRRRRRRRGGQ**GLTIYF**YN | Q44-N52 | 20 |
| #2-D-1-1 | RRRRRRRRGGQ**GLTIYF**Y | Q44-Y51 | 21 |
| #2-D-1-2 | RRRRRRRRGG**GLTIYFY**N | G45-N52 | 22 |
| #2-D-1-3 | RRRRRRRRGGQ**GLTIY** | Q44-Y49 | 23 |
| #2-D-1-4 | RRRRRRRRGG**GLTIYF**Y | G45-Y51 | 24 |
| #2-D-1-1A | RRRRRRRRGGQ**GLTI**AFY | Y49 in Q44-Y51 is substituted with A | 25 |
| #2-D-1-1B | RRRRRRRRGGQ**GLTIYF**A | Y51 in Q44-Y51 is substituted with A | 26 |
| #6-A | RRRRRRRRGG**VVELRVP**TDLTLLPGHLYMM | V116-M135 | 27 |
| #6-B | RRRRRRRRGGIL**TVVELRVP** | 1113-P122 | 28 |
| #6-A-1 | RRRRRRRRGGVPTDLTLLPG | V121-G130 | 29 |
| #6-A-2 | RRRRRRRRGG**VVELRVP** | V116-P122 | 30 |
| #7-A | RRRRRRRRGGLTLLPGHLYMM | L125-M135 | 31 |

### Example 2 Inhibitory effect on proliferation of prostate cancer cell line

Human prostate cancer cell line DU145 was cultured in DMEM containing 10% fetal cattle serum (FCS) at 37°C and 5% CO₂ with passaging. DU145 cells were seeded into 96-well plates at 5000 cells/well, then cultured at 37°C for 24 hours, and then further cultured for 48 hours in 10% FCS-containing DMEM that contains each of the synthesized peptides of Example 1 at a final concentration of 1 µM, 10 µM, 50 µM or 100 µM. The medium was discarded after culturing, and then 25 µL of 50-fold diluted solution of CellTiter Glo Reagent (Promega) (diluted with PBS (-) containing 0.1% NP-40) was added to each well. Next, the resulting solution was shaken at room temperature for 10 minutes, and then luminescence level was measured with a plate reader (Infinite M200, Tecan). A relative luminescence level of each synthetic peptide was calculated when the average luminescence level measured for control cells cultured in 10% FCS-containing DMEM without any synthetic peptide was set to 100, and was used as an index of cell proliferation.

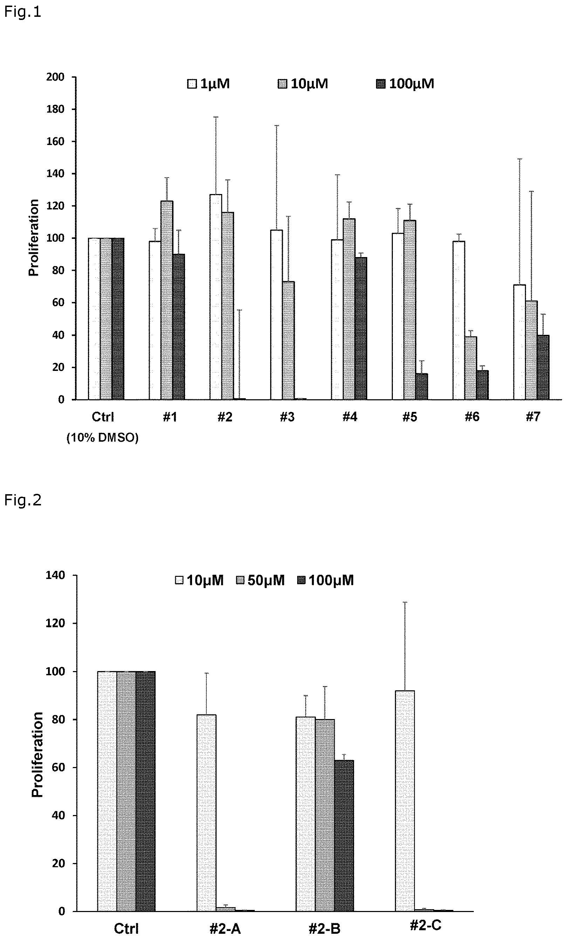

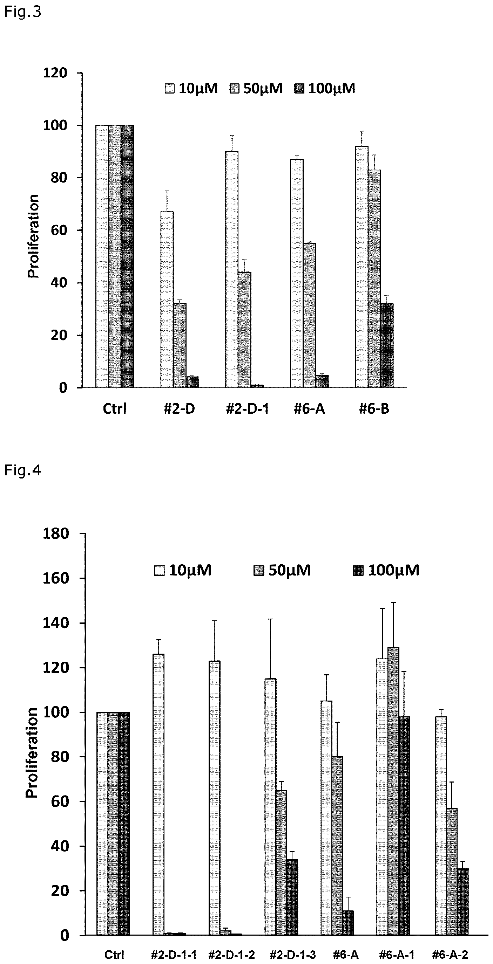

Figs. 1 to 5 show the cell proliferation with addition of each synthetic peptide, when the cell proliferation without addition of any synthetic peptide is set to 100. The peptides #2, #2-A, #2-C, #2-D, #2-D-1, #2-D-1-1, #2-D-1-2, #2-D-1-3, #2-D-1-4, #2-D-1-1A and #2-D-1-1B, which contain the amino acid sequence GLTI (SEQ ID NO: 1), reduced the proliferation of the DU145 cells to less than 40 at a concentration of 100 µM. In a similar manner, the peptides #6, #6-A, #6-B and #6-A-2, which contain the amino acid sequence VVELRVP (SEQ ID NO: 2), reduced the proliferation of the DU145 cells to less than 40 at a concentration of 100 µM.

### Example 3 Inhibitory effect on proliferation of various cancer cell lines

The inhibitory activity of the peptide #2-D-1-4, which was confirmed effective in Example 2, against the human cervical cancer cell line HeLa, the human liver cancer cell line Hep3B, the human lung cancer cell line A549, the human glioma cell line U251, the human melanoma cell line A375, the human pancreatic cancer cell line BxPC3, and the human breast cancer cell line T47D, in the same manner as in Example 2. T47D cells were proliferated slowly and therefore cultured for 96 hours in the presence of the synthetic peptide before evaluation of proliferation.

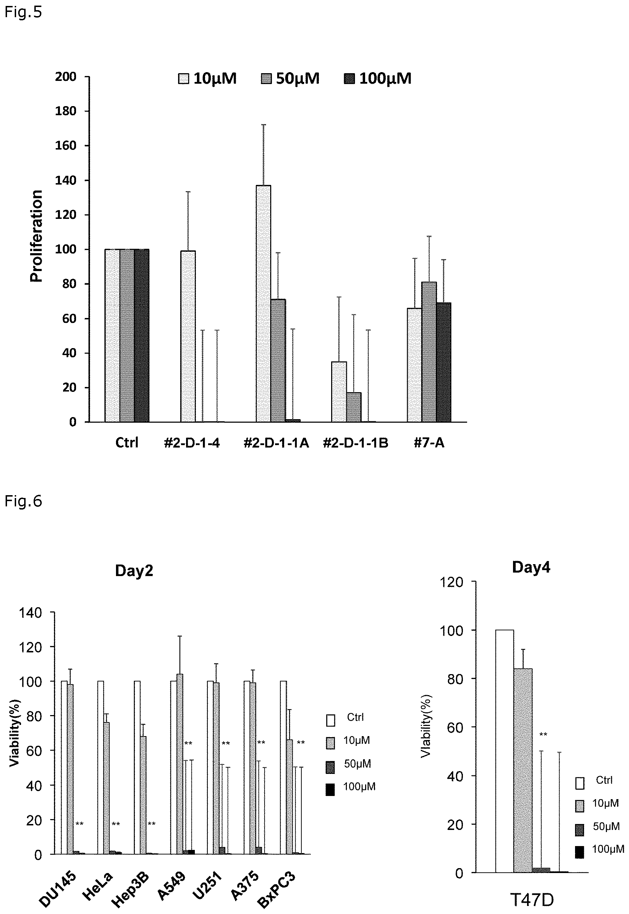

Fig. 6 shows the results. The peptide #2-D-1-4 exhibited the activity to inhibit proliferation of all tested cell lines.

### Example 4 Inhibitory effects of synthetic peptides with addition of various cell-penetrating peptides on proliferation of tumor cells

The peptides listed in Table 2 below were designed and evaluated for their inhibitory activities against proliferation of DU145 cells in the same way as in Example 2. Each of the peptides #T-2-D-1-4, #H-2-D-1-4 and #F-2-D-1-4 is a peptide in which the octaarginine sequence of the peptide #2-D-1-4 is substituted with an amino acid sequence of another cell-penetrating peptide, and each has, from the N-terminal side, an amino acid sequence of TAT peptide, HTLV-II-Rex or FHV coat (35-49), a linker sequence of one Gly residue, and an amino acid sequence corresponding to G45-Y51 of the PH domain. The peptides #R8(-)-2-D-1, #R8(-)-2-D-1-4 and #R8(-)-6-A-2 are peptides with the octaarginine sequence and the linker sequence removed from the peptides #2-D-1, #2-D-1-4 and #6-A-2, respectively.

**[Table 2]**

| Peptide No. | Amino Acid Sequence | SEQ ID NO: |
|---|---|---|
| #T-2-D-1-4 | GRKKRRQRRRPPQGGLTIYFY | 32 |
| #H-2-D-1-4 | TRRQRTRRARRNRGGLTIYFY | 33 |
| #F-2-D-1-4 | RRRRNRTRRNRRRVRGGLTIYFY | 34 |
| #R8(-)-2-D-1 | QGLTIYFYN | 35 |
| #R8(-)-2-D-1-4 | GLTIYFY | 36 |
| #R8(-)-6-A-2 | VVELRVP | 37 |

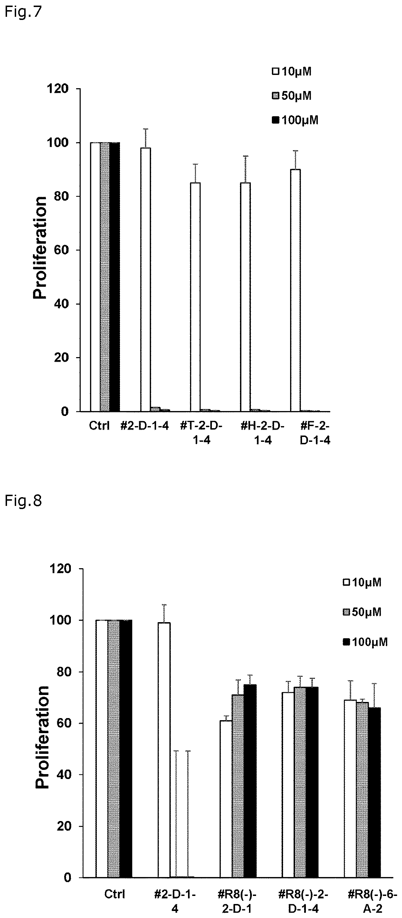

Figs. 7 and 8 show the results. The peptides #T-2-D-1-4, #H-2-D-1-4 and #F-2-D-1-4, each of which a different cell-penetrating peptide is added, all exhibited the same extent of inhibitory activity against cell proliferation as that of the peptide #2-D-1-4. Meanwhile, none of the peptides #R8(-)-2-D-1, #R8(-)-2-D-1-4 and #R8(-)-6-A-2 without the cell-penetrating peptide exhibited the inhibitory activity against cell proliferation. These results demonstrated that inhibition of DU145 cell proliferation by the synthetic peptides required incorporation of the peptides into the cells, and that the synthetic peptides to which cell-penetrating peptides were added exerted the inhibitory activity against cell proliferation regardless of the amino acid sequence of the cell-penetrating peptides.

### Example 5 Inhibitory effects of amino acid substitutions of #2-D-1-4 on proliferation of tumor cells

The peptides listed in Table 3 below were designed and evaluated for their inhibitory activities against proliferation of DU145 cells, in the same way as in Example 2 except that the final concentrations of the synthetic peptides were set to 3 µM, 10 µM and 30 µM. The peptides #2-D-1-4-1A to #2-D-1-4-7A are peptides in which GLTIYFY (SEQ ID NO: 36) of peptide #2-D-1-4 was substituted with Ala one by one from the N-terminal side.

**[Table 3]**

| Peptide No. | Amino Acid Sequence | SEQ ID NO: |
|---|---|---|
| #2-D-1-4 | RRRRRRRRGG GLTIYFY | 24 |
| #2-D-1-4-1A | RRRRRRRRGG ALTIYFY | 39 |
| #2-D-1-4-2A | RRRRRRRRGG GATIYFY | 40 |
| #2-D-1-4-3A | RRRRRRRRGG GLAIYFY | 41 |
| #2-D-1-4-4A | RRRRRRRRGG GLTAYFY | 42 |
| #2-D-1-4-5A | RRRRRRRRGG GLTIAFY | 43 |
| #2-D-1-4-6A | RRRRRRRRGG GLTIYAY | 44 |
| #2-D-1-4-7A | RRRRRRRRGG GLTIYFA | 45 |

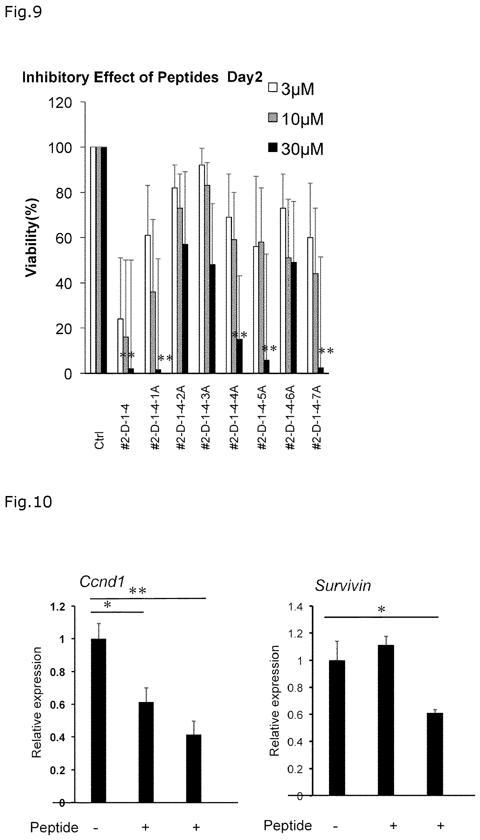

Fig. 9 shows the cell proliferation with addition of each synthetic peptide, when the cell proliferation without addition of any synthetic peptide is set to 100. Among the synthetic peptides listed in Table 3, #2-D-1-4-2A, #2-D-1-4-3A and #2-D-1-4-6A showed cell proliferation of approximately 50% at a final concentration of 30 µM, whereas #2-D-1-4-1A, #2-D-1-4-4A, #2-D-1-4-5A and #2-D-1-4-7A reduced cell proliferation to 20% or less at a final concentration of 30 µM. These results demonstrate that the 2nd residue L, the 3rd residue T, the 5th residue F in the amino acid sequence GLTIYFY are important amino acid residues for inhibitory activity against cell proliferation, whereas substitutions of amino acid residues at other positions can be tolerated. Accordingly, peptides containing the amino acid sequence represented by L-T-Xaa1-Xaa2-F (Xaa1 and Xaa2 each represent any amino acid residue) (SEQ ID NO: 38) are expected to have an inhibitory activity against proliferation of tumor cells.

### Example 6 Effect on expression of genes associated with cell proliferation and survival

DU145 cells, cultured in DMEM containing 10% fetal cattle serum (FCS) at 37°C and 5% CO₂ with passage, were seeded into 6-well plates at 5.0 × 10⁵ cells/well, and then cultured at 37°C for 24 hours. The cells were incubated for 2 hours in serum-free DMEM. Then, the synthetic peptide #2-D-1-4 (final concentration 10 µM) was added, and cells were collected 15 minutes later and 30 minutes later. Cells without addition of any synthetic peptide were prepared in the same way.

Total RNA was extracted from the cells with use of TRIzol (Thermo Fisher Scientific). cDNA was synthesized from the total RNA with use of ReverTra Ace (TOYOBO) and random hexamer primers. Real-time PCR was carried out with the following primer sets and MX3000P (Agilent Technologies, Inc.) to measure the relative gene expression levels of Cyclin D1 (Ccnd1), which is associated with cell proliferation, and Survivin, which is associated with cancer cell survival.
Ccnd1 forward primer 5'-gatgccaacctcctcaacga-3' (SEQ ID NO: 46)
Ccnd1 reverse primer 5'-cacttctgttcctcgcagacc-3' (SEQ ID NO: 47)
Survivin forward primer 5'-ggaccaccgcatctctacat-3' (SEQ ID NO: 48)
Survivin reverse primer 5'-gacagaaaggaaagcgcaac-3' (SEQ ID NO: 49)
GAPDH forward primer 5'-gaaatcccatcaccatcttccagg-3' (SEQ ID NO: 50)
GAPDH reverse primer 5'-cagtagaggcagggatgatgttc-3' (SEQ ID NO: 51)

The addition of the synthetic peptide #2-D-1-4 reduced the gene expression levels of Ccnd1 and Survivin (Fig. 10), demonstrating that the synthetic peptide #2-D-1-4 exhibits an inhibitory activity on the expression of genes associated with cancer.

### Example 7 Effect on EGFR/STAP-2 interaction

A vector expressing human STAP-2 with glutathione S-transferase (GST) added (GST-STAP-2) was prepared by inserting the DNA sequence encoding human STAP-2 (NCBI Reference Sequence: NP_001013863.1, SEQ ID NO: 5) into pEBG vector. A vector expressing human EGFR with human influenza virus hemagglutinin (HA) protein added (EGFR-HA) was prepared by inserting the HA tag sequence and the sequence encoding human EGFR (GenBank: AAH94761.1, SEQ ID NO: 52) into pCIneo (Promega).

HEK293T cells were cultured to semiconfluency in 60 mm dishes and then transfected with EGFR-HA expression vector alone or combination of EGFR-HA expression vector and GST-STAP-2 expression vector using Lipofectamine 2000. After cultured for 48 hours, cells were collected and lysed with lysis buffer (50 mM Tris-HCl, pH 8.0, 150 mM NaCl, 1% Nonidet P-40, protease inhibitor mixture (Sigma)). The synthetic peptide #2-D-1-4 (final concentration 1 µM or 50 µM) was added to the resulting lysate, and incubated at 4°C for 1 hour. Then, glutathione beads (Glutathione Sepharose 4B, GE healthcare) were added to the lysate and incubated at 4°C for 1.5 hours to pull down the GST-added protein in the lysate. After washing the beads, 150 mM reduced glutathione (pH 8) beads were added and incubated at 4°C for 15 minutes to elute the bound product from the beads. Then, the eluate was separated by SDS-PAGE, followed by immunoblotting using anti-HA antibody (HA-7, Sigma) and anti-GST antibody (1-109, Santacruz).

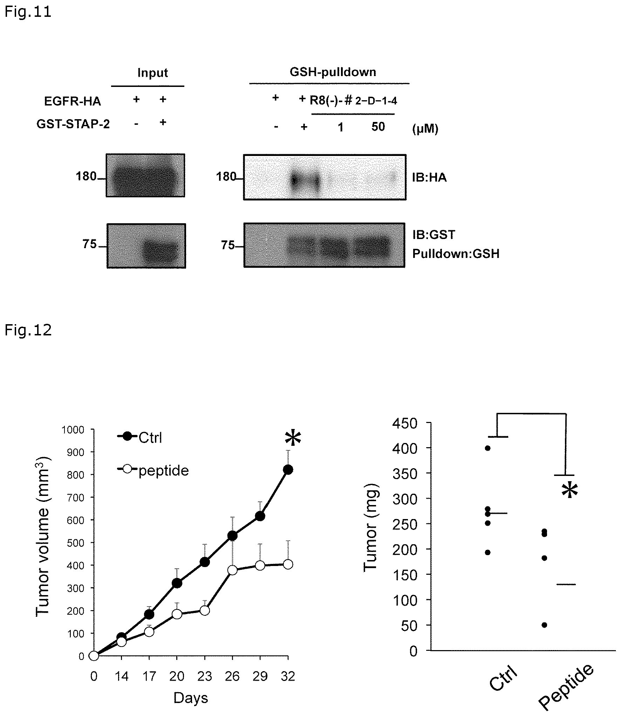

EGFR-HA was detected in the fraction obtained by pulling down the lysate of cells co-expressing EGFR-HA and GST-STAP-2 (Fig. 11, second leftmost lane in the upper right image) with glutathione, indicating the presence of a complex between EGFR-HA and GST-STAP-2. The complex was not detected when being together with the synthetic peptide (Fig. 11, two lanes on the right side of the upper right image). These results demonstrated that the synthetic peptide #2-D-1-4 had an inhibitory effect on the binding between EGFR and STAP-2.

### Example 8 Antitumor effect in tumor-bearing model

DU145 cells (5.0 × 10⁶ cells) were injected subcutaneously into right flank of BALB/c nude mice (female, 4 weeks old). On days 14, 17 and 20 after implantation, solvent (5% DMSO, control) or the synthetic peptide #2-D-1-4 (2 mg/body) was intratumorally administered. Tumor diameters were measured with calipers every 3 days starting on day 14 after implantation, and tumor volumes (longer diameter × shorter diameter × shorter diameter/2) were calculated. The mice were euthanized on day 32 after implantation, then tumor mass were collected and their weights were measured.

The synthetic peptide-administered group exhibited the inhibition of increase in tumor volume and significant reduction of final weight of the tumor mass, compared to the control (Fig. 12). These results demonstrated that the synthetic peptide #2-D-1-4 exhibited an antitumor effect in vivo.

### [Sequence Listing Free Text]

SEQ ID NO: 1 Amino acid sequence contained in a peptide that has activity to inhibit tumor cell proliferation and that has activity to inhibit binding between EGFR and STAP-2
SEQ ID NO: 2 Amino acid sequence contained in a peptide that has activity to inhibit tumor cell proliferation and that has activity to inhibit binding between EGFR and STAP-2
SEQ ID NO: 3 Amino acid sequence contained in a peptide that has activity to inhibit tumor cell proliferation and that has activity to inhibit binding between EGFR and STAP-2
SEQ ID NO: 4 Amino acid sequence contained in a peptide that has activity to inhibit tumor cell proliferation and that has activity to inhibit binding between EGFR and STAP-2
SEQ ID NO: 5 Amino acid sequence of STAP-2
SEQ ID NO: 6 Amino acid sequence of cell-penetrating peptide (TAT peptide)
SEQ ID NO: 7 Amino acid sequence of cell-penetrating peptide (HTLV-II-Rex)
SEQ ID NO: 8 Amino acid sequence of cell-penetrating peptide (FHV coat (35-49))
SEQ ID NO: 9 Amino acid sequence of synthetic peptide #1
SEQ ID NO: 10 Amino acid sequence of synthetic peptide #2
SEQ ID NO: 11 Amino acid sequence of synthetic peptide #3
SEQ ID NO: 12 Amino acid sequence of synthetic peptide #4
SEQ ID NO: 13 Amino acid sequence of synthetic peptide #5
SEQ ID NO: 14 Amino acid sequence of synthetic peptide #6
SEQ ID NO: 15 Amino acid sequence of synthetic peptide #7
SEQ ID NO: 16 Amino acid sequence of synthetic peptide #2-A
SEQ ID NO: 17 Amino acid sequence of synthetic peptide #2-B
SEQ ID NO: 18 Amino acid sequence of synthetic peptide #2-C
SEQ ID NO: 19 Amino acid sequence of synthetic peptide #2-D
SEQ ID NO: 20 Amino acid sequence of synthetic peptide #2-D-1
SEQ ID NO: 21 Amino acid sequence of synthetic peptide #2-D-1-1
SEQ ID NO: 22 Amino acid sequence of synthetic peptide #2-D-1-2
SEQ ID NO: 23 Amino acid sequence of synthetic peptide #2-D-1-3
SEQ ID NO: 24 Amino acid sequence of synthetic peptide #2-D-1-4
SEQ ID NO: 25 Amino acid sequence of synthetic peptide #2-D-1-1A
SEQ ID NO: 26 Amino acid sequence of synthetic peptide #2-D-1-1B
SEQ ID NO: 27 Amino acid sequence of synthetic peptide #6-A
SEQ ID NO: 28 Amino acid sequence of synthetic peptide #6-B
SEQ ID NO: 29 Amino acid sequence of synthetic peptide #6-A-1
SEQ ID NO: 30 Amino acid sequence of synthetic peptide #6-A-2
SEQ ID NO: 31 Amino acid sequence of synthetic peptide #7-A
SEQ ID NO: 32 Amino acid sequence of synthetic peptide #T-2-D-1-4
SEQ ID NO: 33 Amino acid sequence of synthetic peptide #H-2-D-1-4
SEQ ID NO: 34 Amino acid sequence of synthetic peptide #F-2-D-1-4
SEQ ID NO: 35 Amino acid sequence of synthetic peptide #R8(-)-2-D-1
SEQ ID NO: 36 Amino acid sequence of synthetic peptide #R8(-)-2-D-1-4
SEQ ID NO: 37 Amino acid sequence of synthetic peptide #R8(-)-6-A-2
SEQ ID NO: 38 Amino acid sequence contained in a peptide that has activity to inhibit tumor cell proliferation and that has activity to inhibit binding between EGFR and STAP-2
SEQ ID NO: 39 Amino acid sequence of synthetic peptide #2-D-1-4-1A
SEQ ID NO: 40 Amino acid sequence of synthetic peptide #2-D-1-4-2A
SEQ ID NO: 41 Amino acid sequence of synthetic peptide #2-D-1-4-3A
SEQ ID NO: 42 Amino acid sequence of synthetic peptide #2-D-1-4-4A
SEQ ID NO: 43 Amino acid sequence of synthetic peptide #2-D-1-4-5A
SEQ ID NO: 44 Amino acid sequence of synthetic peptide #2-D-1-4-6A
SEQ ID NO: 45 Amino acid sequence of synthetic peptide #2-D-1-4-7A
SEQ ID NO: 46 Nucleotide sequence of forward primer for Ccnd1 amplification
SEQ ID NO: 47 Nucleotide sequence of reverse primer for Ccnd1 amplification
SEQ ID NO: 48 Nucleotide sequence of forward primer for Survivin amplification
SEQ ID NO: 49 Nucleotide sequence of reverse primer for Survivin amplification
SEQ ID NO: 50 Nucleotide sequence of forward primer for GAPDH amplification
SEQ ID NO: 51 Nucleotide sequence of reverse primer for GAPDH amplification
SEQ ID NO: 52 Amino acid sequence of human EGFR

## Claims

1. A peptide consisting of an amino acid sequence with 20 or fewer residues containing at least one amino acid sequence selected from the group consisting of the amino acid sequence represented in SEQ ID NO: 1, the amino acid sequence represented in SEQ ID NO: 2, and the amino acid sequence represented in SEQ ID NO: 38, the peptide having an activity to inhibit proliferation of a tumor cell.

2. The peptide according to claim 1, consisting of an amino acid sequence with 20 or fewer residues containing the amino acid sequence represented in SEQ ID NO: 3.

3. The peptide according to claim 1 or 2, consisting of an amino acid sequence with 20 or fewer residues containing the amino acid sequence represented in SEQ ID NO: 4.

4. The peptide according to claim 1, wherein 3rd amino acid residue Xaa is isoleucine, alanine, leucine or valine, and 4th amino acid residue Xaa is tyrosine, alanine or phenylalanine, in the amino acid sequence represented in SEQ ID NO: 38.

5. A peptide consisting of an amino acid sequence with 20 or fewer residues containing at least one amino acid sequence selected from the group consisting of the amino acid sequence represented in SEQ ID NO: 1, the amino acid sequence represented in SEQ ID NO: 2 and the amino acid sequence represented in SEQ ID NO: 38, the peptide having an activity to inhibit binding between an epidermal growth factor receptor and a signal-transducing adaptor family member-2 (STAP-2).

6. The peptide according to claim 5, consisting of an amino acid sequence with 20 or fewer residues containing the amino acid sequence represented in SEQ ID NO: 3.

7. The peptide according to claim 5 or 6, consisting of an amino acid sequence with 20 or fewer residues containing the amino acid sequence represented in SEQ ID NO: 4.

8. The peptide according to claim 5, wherein 3rd amino acid residue Xaa is isoleucine, alanine, leucine or valine, and 4th amino acid residue Xaa is tyrosine, alanine or phenylalanine, in the amino acid sequence represented in SEQ ID NO: 38.

9. A peptide with a cell-penetrating peptide added to a terminus of the peptide according to any one of claims 1 to 8, with or without a linker sequence interposed between the cell-penetrating peptide and the terminus.

10. The peptide according to claim 9, wherein the linker sequence consists of one to five glycine(s).

11. The peptide according to any one of claims 1 to 10, comprising one or more chemically modified amino acid residue(s).

12. A nucleic acid encoding the peptide according to any one of claims 1 to 11.

13. A pharmaceutical composition for treating a cancer, the pharmaceutical composition comprising the peptide according to any one of claims 1 to 11 or the nucleic acid according to claim 12.

14. The pharmaceutical composition according to claim 13, for treating prostate cancer, cervical cancer, liver cancer, lung cancer, glioma, melanoma, pancreatic cancer or breast cancer.

15. A composition for inhibition of binding between an epidermal growth factor receptor and a signal-transducing adaptor family member-2 (STAP-2), the composition comprising the peptide according to any one of claims 1 to 11 or the nucleic acid according to claim 12.
